# EUROPEAN PATENT SPECIFICATION

(11) **EP 2 558 455 B1**
(45) Date of publication and mention of the grant of the patent: **09.08.2017**
(21) Application number: 11714279.4
(22) Date of filing: 13.04.2011
(51) Int. Cl.: C07D 333/20, A61P 13/02, A61P 25/24, A61K 31/381

(54) **SYNTHESIS OF DULOXETINE AND/OR PHARMACEUTICALLY ACCEPTABLE SALTS THEREOF**
SYNTHESE VON DULOXETIN- UND/ODER PHARMAZEUTISCH AKZEPTABLEN SALZEN DARAUS
SYNTHÈSE DE DULOXÉTINE ET/OU DE SES SELS PHARMACEUTIQUEMENT ACCEPTABLES DE CELLE-CI

(30) Priority: 13.04.2010 SI 201000124
(43) Date of publication of application: 20.02.2013
(73) Proprietor: KRKA, D.D., Novo Mesto, 8501 Novo mesto (SI)
(72) Inventor: BOMBEK, Sergeja, 8501 Novo mesto (SI); MERSLAVIC, Marjo, 8501 Novo Mesto (SI); BENKIC, Primoz, 1000 Ljubljana (SI); ZAJC, Natalija, 8501 Novo Mesto (SI); VRECER, Franc, 8501 Novo Mesto (SI)
(74) Representative: Hoffmann Eitle
(86) International application number: PCT/EP2011/055805
(87) International publication number: WO 2011/128370

(56) References cited:
- WO-A2-2010/003942
- US-A- 5 362 886
- BERGE S M ET AL: "PHARMACEUTICALS SALTS", JOURNAL OF PHARMACEUTICAL SCIENCES, AMERICAN PHARMACEUTICAL ASSOCIATION, WASHINGTON, US, vol. 66, no. 1, 1 January 1977 (1977-01-01), pages 1-19, XP000562636, ISSN: 0022-3549, DOI: 10.1002/JPS.2600660104

## Description

### Technical Field

The invention belongs to the field of organic chemistry, more precisely of chemical synthesis and deals with an improved process for the synthesis of pharmaceutically acceptable salts of duloxetine . The described process is economically acceptable, environmentally friendly and technologically simple.

### Prior Art

Duloxetine with the chemical name (*S*)-*N*-methyl-3-(1-naphthalenyloxy)-3-(2-thienyl)propanamine is a dual serotonin and norepinephrine inhibitor. Duloxetine in the form of hydrochloride is used in therapy especially as antidepressant and for alleviation of incontinence problems. It can also be prepared in the form of other pharmaceutically acceptable salts, such as oxalic acid salt, maleic acid salt. Duloxetine and pharmaceutically acceptable salts thereof were first described in EP 273658 B1.

Processes for the preparation of duloxetine and/or pharmaceutically acceptable salts thereof are known from literature, they are disclosed in e.g. US 5,023,269 and US 4,956,388, Tetrahedron Letters (1990) 31(49), 7101-7104, Drugs of the Future (2000) 25(9), 907-916, Journal of Labeled Compounds and Radiopharmaceuticals (1995) 36(3), 213-223 and WO2010/003942.

Polymorphic forms of duloxetine chloride are described in WO 2005/019199 and WO 2006/081515.

There is a need for an efficient synthesis of duloxetine and/or pharmaceutically acceptable salts thereof with high chemical and enantiomeric purity.

Therefore, the purpose of the invention is preparation of optically pure pharmaceutically acceptable salts of duloxetine with high chemical and enantiomeric purity and high yield.

This aim is accomplished by the process of synthesis according to the present invention having the following advantages:
- it is performed without intermediate conversion and isolation of (S)-N,N-dimethyl-3-(naphthalen-1-yloxy)-3-(thiophen-2-yl)propan-1-amine oxalate, which in the complete process represents two steps less in a synthesis process,
- it includes a direct entry of a solution of (S)-N,N-dimethyl-3-(naphthalen-1-yloxy)-3-(thiophen-2-yl)propan-1-amine into demethylation step (i. e. "one pot" synthesis);
- it uses organic acids in the precipitation step and optionally in the recrystallization of duloxetine salt step.

### Summary of the Invention

The first subject of the invention is a process for the preparation of chemically and optically pure pharmaceutically acceptable salts of duloxetine, preferably hydrochloride, according to the following process (Scheme 1), as specified in appended claim 1:

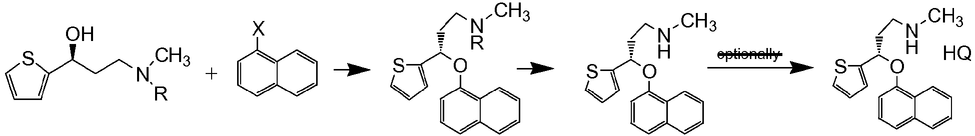

A preferred reaction sequence according to the present invention is shown is in the following Scheme 1': 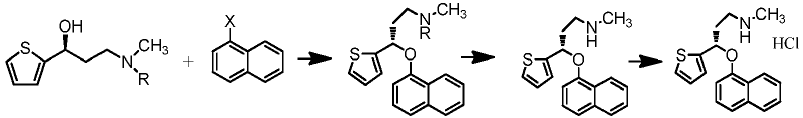 wherein in both schemes R represents H or CH₃, and
wherein X represents a leaving group, preferably halogens F, Cl, Br or I, more preferably F, and
wherein Q represents the anion of a pharmaceutically acceptable salt.

A further subject of the invention is a process for the synthesis of pharmaceutically acceptable salts of duloxetine according to the process of Scheme 1 as specified in appended claim 1, wherein R=H, comprising the following reaction steps:
a) synthesis of duloxetine by a reaction between (S)-3-methyl-amino-1-(2-thienyl)-1-propanol and 1-fluoronaphthalene in the presence of a base, preferably NaH, in polar aprotic solvents such as acetonitrile, dimethyl sulfoxide, dimethyl formamide, dimethyl acetamide, N-methyl pyrrolidone and mixtures thereof, preferably dimethyl sulfoxide,
b) formation of a duloxetine salt with an acid, preferably in the form of hydrochloride, in organic solvents, sometimes referred to as "extraction organic solvents", selected from the group comprising ethers, esters, ketones, hydrocarbons, preferably isopropyl acetate, and in the presence of an organic acid, preferably selected from the group comprising formic acid, acetic acid, propanoic acid, butanoic acid or any mixtures thereof, more preferably acetic acid,
c) recrystallization of a duloxetine salt, preferably in the form of hydrochloride, in a mixture of organic solvents, selected from the group comprising ethers, esters, alcohols, hydrocarbons, preferably isopropanol, and optionally in the presence of an organic acid, preferably selected from the group comprising formic acid, acetic acid, propanoic acid, butanoic acid or any mixtures thereof, more preferably acetic acid.

A further subject of the invention is a process for the synthesis of pharmaceutically acceptable salts of duloxetine according to the process of Scheme 1 as specified in appended claim 1, wherein R=CH₃, which may comprise the following reaction steps:
a) synthesis of (S)-N,N-dimethyl-3-(naphthalen-1-yloxy)-3-(thiophen-2-yl)propan-1-amine by a reaction between (S)-3-methyl-amino-1-(2-thienyl)-1-propanol and 1-fluoronaphthalene in the presence of a base, preferably NaH, in polar aprotic solvents such as acetonitrile, dimethyl sulfoxide, dimethyl formamide, dimethyl acetamide, N-methyl pyrrolidone and mixtures thereof, preferably dimethyl sulfoxide,
b) demethylation of the solution of (S)-N,N-dimethyl-3-(naphthalen-1-yloxy)-3-(thiophen-2-yl)propan-1-amine with chloroformate and subsequent alkaline hydrolysis,
c) formation of a duloxetine salt with an acid, preferably in the form of hydrochloride, in organic solvents, sometimes referred to as "extraction organic solvents", selected from the group comprising ethers, esters, ketones, hydrocarbons, preferably isopropyl acetate, and in the presence of an organic acid, preferably selected from the group comprising formic acid, acetic acid, propanoic acid, butanoic acid or any mixtures thereof, more preferably acetic acid,
d) optionally recrystallization of duloxetine salt, preferably in the form of hydrochloride, in a mixture of organic solvents selected from the group comprising ethers, esters, alcohols, hydrocarbons, preferably isopropanol, and optionally in the presence of an organic acid, preferably selected from the group comprising formic acid, acetic acid, propanoic acid, butanoic acid or any mixtures thereof, more preferably acetic acid.

An essential feature of the invention is as specified in appended claim 1 is the addition of an organic acid in the phase of formation of a duloxetine salt with an acid, preferably in the form of hydrochloride, and optionally in the phase of recrystallization of the formed duloxetine salt, wherein the organic acid is preferably selected from the group comprising formic acid, acetic acid, propanoic acid, butanoic acid or any mixtures thereof, more preferably acetic acid.

A further subject of the invention is a process for the manufacture of a pharmaceutical formulation including a pharmaceutically acceptable salt of duloxetine as specified in appended claim 15, with high chemical and enantiomeric purity.

### Detailed Description of the Invention

The purpose of the invention is a preparation of pharmaceutically acceptable salts of duloxetine with high chemical and enantiomeric purity in a technologically simple and economical manner.

Therefore, the first subject of the invention is a process for the preparation of chemically and optically pure pharmaceutically acceptable salts of duloxetine, preferably in the form of hydrochloride, according to the process as shown in Scheme 1, as specified in appended claim 1.

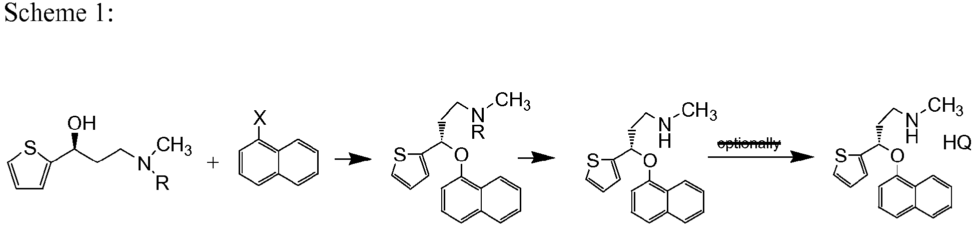

A preferred reaction sequence according to the present invention is shown is in the following Scheme 1': 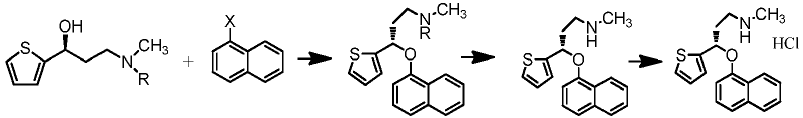 wherein R represents H or CH₃, and
wherein X represents a leaving group, preferably a halogen atom selected from F, Cl, Br or I, more preferably F, and
wherein Q represents the anion of a pharmaceutically acceptable salt.

In more detail, a further subject of the invention is a process for the synthesis of pharmaceutically acceptable salts of duloxetine according to the process of Scheme 1 as specified in appended claim 1, wherein R=H, which may comprise the following reaction steps:
a) synthesis of duloxetine by a reaction between (S)-3-methyl-amino-1-(2-thienyl)-1-propanol and 1-fluoronaphthalene in the presence of a base, preferably NaH, in polar aprotic solvents such as acetonitrile, dimethyl sulfoxide, dimethyl formamide, dimethyl acetamide N-methyl pyrrolidone and mixtures thereof, preferably dimethyl sulfoxide,
b) formation of a duloxetine salt with an acid, preferably in the form of hydrochloride, in organic solvents, sometimes referred to as "extraction organic solvents", selected from the group comprising ethers, esters, ketones, hydrocarbons, preferably isopropyl acetate, and in the presence of an organic acid, preferably selected from the group comprising formic acid, acetic acid, propanoic acid, butanoic acid or any mixtures thereof, more preferably acetic acid,
c) optionally recrystallization of a duloxetine salt, preferably in the form of hydrochloride, in an organic solvent, selected from the group comprising ethers, esters, alcohols, hydrocarbons, preferably isopropanol and mixtures thereof, and optionally in the presence of an organic acid, preferably selected from the group comprising formic acid, acetic acid, propanoic acid, butanoic acid or any mixtures thereof, more preferably acetic acid.

Formation of a duloxetine salt, preferably in the form of hydrochloride, is preferably carried out with an excess of an acid, such as in the case of hydrochloride, where the excess is up to 1.5 equivalents, preferably up to 1.1 equivalents.

Duloxetine salt, preferably in the form of hydrochloride, obtained according to the process of the present invention in step b) has a chemical purity of more than 99.5 %, preferably more than 99.8 %, and an enantiomeric purity of more than 98 %, preferably more than 99.0 %. At the same time, the yield of the step b) is surprisingly high, namely between 80 % and 90 %, typically between 82 % and 84 %.

Duloxetine salt, preferably in the form of hydrochloride, obtained according to the process of the present invention in step c) has a chemical purity of more than 99.7 %, preferably more than 99.9 % and an enantiomeric purity of more than 99.7 %, preferably 100 %. Yield of step c) is between 90 % and 95 %, preferably between 93 % and 95 %.

The reaction between (S)-3-methyl-amino-1-(2-thienyl)-1-propanol and 1-fluoronaphthalene is performed at a temperature between 30 °C and 90 °C, preferably between 35 °C and 70 °C, more preferably between 40 °C and 50 °C.

The synthesis of the starting compound (S)-3-methyl-amino-1-(2-thienyl)-1-propanol is known from literature and is described e. g. in WO 0061540, WO 03062219, WO 03/097632, WO 2004013123, WO2004016603, WO2004020389, US 7119211, WO2004031168, EP1411045, WO2004065376, WO2005021527, WO 2005073215, WO2005080370, WO 2005085192, EP2060559, WO2010003942.

A further subject of the invention is a process for the synthesis of pharmaceutically acceptable salts of duloxetine according to the process of Scheme 1, wherein R=CH₃, which may comprise the following reaction steps:
a) synthesis of (S)-N,N-dimethyl-3-(naphthalen-1-yloxy)-3-(thiophen-2-yl)propan-1-amine by a reaction between (S)-3-dimethyl-amino-1-(2-thienyl)-1-propanol and 1-fluoronaphthalene in the presence of a base, preferably NaH, in polar aprotic solvents such as acetonitrile, dimethyl sulfoxide, dimethyl formamide, dimethyl acetamide, N-methyl pyrrolidone and mixtures thereof, preferably dimethyl sulfoxide,
b) demethylation of the obtained (S)-N,N-dimethyl-3-(naphthalen-1-yloxy)-3-(thiophen-2-yl)propan-1-amine with chloroformate, such as alkyl or phenyl chloroformate,
c) formation of a duloxetine salt with an acid, preferably in the form of hydrochloride, in organic solvents, sometimes referred to as "extraction organic solvents", selected from the group comprising ethers, esters, ketones, hydrocarbons, preferably isopropyl acetate, and in the presence of an organic acid, preferably selected from the group comprising formic acid, acetic acid, propanoic acid, butanoic acid or any mixtures thereof, more preferably acetic acid,
d) optionally recrystallization of a duloxetine salt, preferably in the form of hydrochloride, in an organic solvent, selected from the group comprising ethers, esters, alcohols, hydrocarbons, preferably isopropanol and mixtures thereof, and optionally in the presence of an organic acid, preferably selected from the group comprising formic acid, acetic acid, propanoic acid, butanoic acid or any mixtures thereof, more preferably acetic acid.

The reaction between (S)-3-dimethyl-amino-1-(2-thienyl)-1-propanol and 1-fluoronaphthalene is performed at a temperature between 30 °C and 90 °C, preferably between 40 °C and 70 °C, more preferably between 55 °C and 65 °C.

Preferably, the formed product is subjected to the reaction of demethylation in step b) without purification and without formation and isolation of (S)-N,N-dimethyl-3-(naphthalen-1-yloxy)-3-(thiophen-2-yl)propan-1-amine oxalate.

Formation of a duloxetine salt, preferably in the form of hydrochloride, is preferably carried out with an excess of an acid, such as in the case of hydrochloride the excess is up to 1.5 equivalents, preferably up to 1.1 equivalents.

Duloxetine in the form of a salt, preferably duloxetine chloride, obtained according to the process of the present invention in step c) has chemical purity of more than 99.0 %, preferably more than 99.7 %, and enantiomeric purity of more than 98 %, preferably more than 99.0 %. At the same time, the yield of the step c) is surprisingly high, namely between 70 % and 90 %, typically between 73 % and 75 %.

Duloxetine salt, preferably in the form of hydrochloride, obtained according to the process of the present invention in step d) has chemical purity of more than 99.5 %, preferably more than 99.9 %, and enantiomeric purity of more than 99.7 %, preferably 100 %. Yield of step d) is between 90 % and 95 %, typically between 92 % and 93 %.

Preferably the synthesis in steps a), b) and optionally c) is performed without isolation of intermediate products (i. e. "one pot synthesis").

The synthesis of the starting compound (S)-3-(dimethylamino)-1-(thiophen-2-yl) propanol is known from literature and is described e. g. in WO 03/097632, WO 0061540, WO2009086283.

Further described herein is a duloxetine salt obtained according to the process of the present invention, with high enantiomeric purity of more than 99.7 %, preferably 100 %, and high chemical purity of more than 99.5 %, preferably more than 99.9 %.

Prior to its use in the preparation of a pharmaceutical form, the medicinal active ingredient can be milled and thus the size of the particles is reduced. Any process and equipment for milling of pharmaceutical substances known in the literature can be used. Duloxetine hydrochloride typically has an average diameter smaller than 40 µm and d90 smaller than 55 µm, preferably has an average diameter smaller than 30 µm and d90 smaller than 45 µm, more preferably an average diameter smaller than 20 µm and d90 smaller than 35 µm. Particle size was measured by laser diffraction method (Malvern Mastersizer 2000 instrument) in a vegetable oil without prior application of ultrasound. Absorption factor (A=1) was used.

Duloxetine chloride obtained according to the process of the present invention is typically in polymorphic form A, as described in WO 2006/081515.

An essential feature of the invention as specified in appended claim 1 is addition of an organic acid in the phase of formation of duloxetine salt with an acid, preferably in the form of hydrochloride, and optionally in the phase of recrystallization of the formed duloxetine salt, wherein an organic acid is preferably selected from the group comprising formic acid, acetic acid, propanoic acid, butanoic acid or any mixtures thereof, more preferably acetic acid. If the organic solvent is used in the recrystallization of the duloxetine salt, it is preferably added prior to the addition of the recrystallization solvent.

Essential is the addition of an organic acid in the phase of formation of a duloxetine salt with an acid, preferably in the form of hydrochloride, which surprisingly reflects in high chemical and enantiomeric purity of the obtained crude product. The addition of an organic acid at the recrystallization step is also preferred because it gives rise to improved enantiomeric and chemical purity.

In has surprisingly been found out that the addition of an organic acid has several advantages such as:
- significant reduction in the colouring of the obtained product, which is a consequence of the presence of oxidative decomposition products due to oxidative properties of polar aprotic solvents, especially dimethyl sulfoxide, which are used in step a) of the synthesis process of the present invention,
- removal of acidic decomposition impurity with the chemical name N-methyl-3-(thiophen-2-yl)propyl-2-en-1-amine to less than 0.10 %, preferably less than 0.05 %,
- isolation of the final product with high enantiomeric purity of more than 99.7 %, preferably 100 %, due to the presence of less R-isomer, and high chemical purity of more than 99.5 %, preferably more than 99.9 %, due to the presence of less impurities such as acidic/alkaline decomposition products such as regioisomer N-methyl-3-(naphthalen-1-yloxy)-3-(thiophen-3-yl)propan-1-amine, non-reacted starting compounds, intermediates of synthesis such as the compound with the chemical name (S)-N,N-dimethyl-3-(naphthalen-1-yloxy)-3-(thiophen-2-yl)propan-1-amine,
- controlled nucleation due to a slower precipitation of the product from an oversaturated mixture of solvents due to increased solubility and stability of the product, at the same time nucleation can be performed also at higher temperatures, such as up to a temperature of 55 °C without formation of additional acidic decomposition products. The formed product instantly nucleates from oversaturated solution in media without an addition of organic acid, which causes adhesion/concreting of the product on the parts of industrial equipment.

In the reaction between 1-fluoronaphthalene and a chiral alcohol in dimethyl sulfoxide at an elevated temperature the formation of an ether bond is accompanied by partial racemisation. The process of using acetic acid in the phase of precipitation of crude duloxetine chloride from an organic solvent, after the reaction with 10 % content of R-enantiomer, enables an isolation of the product with ee 99.0 %, if compared with the process without an addition of organic acid, the isolated product has ee 84.4 % (Examples 3 and 4). In the phase of recrystallization of crude duloxetine chloride from an organic solvent in the presence of acetic acid, and in the presence of 10 % content of R-enantiomer, the process enables an isolation of the product with ee 99.4 %, if compared with the process without an addition of organic acid, the isolated product has ee 97.2 % (Examples 5 and 6).

A further subject of the invention is a process for the manufacture of pharmaceutical formulations including pharmaceutically acceptable salts of duloxetine with high chemical and enantiomeric purity.

Pharmaceutical formulations obtained by the process of the present invention are preferably based upon coated pellets containing pharmaceutically acceptable salts of duloxetine in the core, the core being coated with at least one film coating insoluble in an acidic medium with pH less than 4, preferably with pH less than 3.5, named a gastro-resistant coating. In addition to at least one gastro-resistant coating, the coated pellets containing pharmaceutically acceptable salts of duloxetine can contain additional film coatings, e. g. one or more intermediate coatings, which physically separate the core containing medicinal active ingredient from the gastro-resistant coating, whereby higher stability of the medicinal active ingredient in the final pharmaceutical form is achieved and, if necessary, also outer coating which contributes to the organoleptic appearance of the final pellets such as colour and/or to reduced ability to bind moisture into the coated pellets.

The core containing pharmaceutically acceptable salts of the medicinal active ingredient duloxetine can be in the form of granules, microtablets and/or tablets, preferably in the form of spherical granules such as pellets. The core can be made by the processes known in prior art, such as granulation, including the processes of dry and/or wet granulation, coating or pressing. The maximum average diameter of the core, determined by microscopic analysis, is in the range of 0.1 mm to 7 mm, preferably of 0.2 mm to 5 mm.

The medicinal active ingredient can be distributed in the core homogenously over the entire volume of the pharmaceutical form, such as matrix pharmaceutical form, or it can be present in the form of a coating surrounding the carrier, such as for example granule, pellet or tablet core. In case where the core containing pharmaceutically acceptable salts of duloxetine is present in the form of a matrix pharmaceutical form, this can be in the form of granules, pellets or tablets, including microtablets. The cores in the form of a matrix pharmaceutical form can be made in any manner and with any equipment known in prior art. Some examples of manufacturing are described in continuation:
- Granulate containing pharmaceutically acceptable salts of duloxetine can be made by the processes of dry or wet granulation or melt granulation, whereby the medicinal active ingredient is mixed with at least one excipient selected from the group, which may consist of fillers, binders, disintegrants, surfactants, stabilisers, glidants, lubricants, and the obtained mixture is granulated. In dry granulation processes briquetting or compacting can be used, whereby the obtained compressed material is crushed and sieved in order to obtain granule fraction with the required size of particles.
   In case of wet granulation, a mixture of the medicinal active ingredient and at least one excipient is granulated with a granulating liquid in the form of a dispersion containing water, organic solvents or mixtures thereof as a dispersing agent, and optionally at least one excipient selected from the group, which may comprise binders, surfactants, stabilisers, which is dissolved or suspended in a dispersing agent. Granulation can be performed in fluid bed granulators or high shear mixing granulators, such as high-rotating granulators, a typical representative of which is a high-shear mixer/granulator of the company Collett.
   In case of melt granulation the medicinal active ingredient alone or in a mixture with at least one excipient is granulated with a melt of the excipient, the melting point or softening point of which is under 150 °C, preferably under 100°C.
- Matrix pellets such as cores can be made by the processes known in prior art, such as extrusion and spheronization, direct pelleting of powders in rotating pelletizers, such as high shear mixers/granulators, rotor granulators, or similar processes, whereby the medicinal active ingredient is mixed homogenously with at least one excipient selected from the group that may comprise fillers, binders, disintegrants, surfactants, stabilisers, glidants, lubricants. In case of extrusion and spheronization, a mixture of the medicinal active ingredient and at least one excipient is granulated prior to extrusion by one of the above mentioned processes. A special example of extrusion is melt extrusion, wherein the medicinal active ingredient is mixed with a polymer having the melting point or softening point below 150 °C, preferably below 100 °C, and if necessary, other excipients selected from the group which may comprise fillers, binders, disintegrants, surfactants, stabilisers, glidants or lubricants, then the formed mixture is extruded through an extruder, where the temperature of the mixture is at least 10 °C above the melting point or softening point temperature of the polymer. The obtained extrudates are formed into spherical granules with maximum diameter 0.2 mm to 3 mm, preferably 0.3 mm to 1.5 mm.
- Microtablets or tablets can be prepared by pressing the mixture of pharmaceutically acceptable salts of duloxetine and excipients selected from the group which may comprise fillers, binders, disintegrants, surfactants, stabilisers, glidants, lubricants. Prior to pressing into a tablet the mixture can be partly or completely granulated. The maximum diameter of the manufactured tablet cores is in the range of 1.5 mm to 8 mm, preferably 2 mm to 5 mm.

In a special example the cores represent a coated pharmaceutical form, in which the coating containing a medicinal active ingredient is applied on a carrier, which may be in the form of granules, pellets, microtablets or tablet cores and does not contain a medicinal active ingredient.

Coating can be carried out by the processes and equipment known from prior art, such as fluid-bed coaters or coating drums. In the latter, perforated or non-perforated drums can be used depending on the carrier size.

Coated cores can be prepared in such a manner that onto a carrier (granules, pellets, microtablets or tablets) with a maximum diameter between 0.1 mm and 5 mm and which does not contain a medicinal active ingredient a coating comprising a medicinal active ingredient and at least one excipient selected from the group, which may comprise fillers, binders, disintegrants, stabilizers, surfactants and mixtures thereof, is applied in a suitable coating equipment. Thickness of such coating with the active ingredient is between 10 µm and 300 µm, preferably between 20 µm and 200 µm.

Preferably the cores represent matrix pellets or coated pellets.

The cores containing pharmaceutically acceptable salts of duloxetine, prepared according to the above described processes, are coated with at least one gastro-resistant coating by the processes known in prior art, such as coating in fluid-bed coaters and/or coating drums, whereby coating dispersion is sprayed on cores that either float in a warmed air flow or roll in the drum, whereby simultaneously the evaporation of the solvent and thus drying of the cores are performed. Dispersion for coating with gastro-resistant coating contains in addition to a solvent, selected from the group which may comprise water, pharmaceutically acceptable organic solvents, selected from the group, which may comprise alcohols with 1-4 carbon atoms, ketones or esters and/or mixtures thereof, also a polymer, which is insoluble in acidic medium with pH less than 4 and soluble in a medium with pH of more than 5, such as e. g. hydroxypropyl methylcellulose acetate succinate, hydroxypropyl methylcellulose phthalate, methacrylic acid copolymer and carboxymethylethyl cellulose and if necessary, also other pharmaceutically acceptable excipients selected from the group, which may comprise plasticizers, pigments, colorants, antiadhesives. Thickness of the gastro-resistant coating can be in a range from 10 µm to 150 µm, preferably from 20 µm to 120 µm. To a dispersion for coating with gastro-resistant coating also a plasticizer, most often triethyl citrate, Macrogol, propylene glycol and similar can be added. Its task is to reduce glass transition point temperature of the polymer and thus enabling formation of uniform and coherent gastro-resistant coating. The plasticizer may be added in an amount of up to 30%, preferably 15%, more preferably 10% with regard to the mass of polymer. When carrying out the enteric coating process it is possible to select the process parameters such that desired product characteristics are obtained. The process as such affects the thickness, coherence, integrity and therefore quality and performance of enteric coating.

Between the core and the gastro-resistant coating one or more intermediate film coatings, which dissolve in water in less than 20 minutes, can be applied. Intermediate film coatings consist of at least one water-soluble polymer, selected from the group which may comprise cellulose ethers such as hydroxypropyl methylcellulose, hydroxy propylcellulose, povidone, polyvinyl alcohol and the like. Intermediate coating can be applied by spraying a dispersion, which in addition to water-soluble polymer also contains a solvent and, if necessary, other excipients selected from the group, which may comprise water-soluble fillers, stabilizers, antiadhesives, pigments, colorants, plasticizers. Thickness of the intermediate coating can be between 5 µm and 120 µm, preferably 10 µm to 100 µm.

Optionally, an outer film coating can be applied on the pharmaceutical form containing pharmaceutically acceptable salts of duloxetine and being coated with gastro-resistant form, said film coating allowing colour distinction between pellets and/or reduced possibility of diffusion of moisture and/or oxygen inside of the pharmaceutical form. Film coating can be applied by the processes and in the equipment known form prior art, whereby a dispersion of water-soluble binder and if necessary other pharmaceutically acceptable excipients, selected from the group which may comprise pigments, colorants, antiadhesives, plasticizers and/or fillers, is applied on a pharmaceutical form coated with gastro-resistant coating. In a special embodiment of the present invention, outer film coating can increase the roughness of the surface of pellets, whereby higher ability of pellets for tabletting is achieved. Thickness of the outer film coating is between 5 µm and 100 µm, preferably between 8 µm and 60 µm.

Fillers used in the pharmaceutical formulation obtained according to the present invention are selected from the group of water-soluble pharmaceutical substances, characterized in that 1 g of a substance is dissolved in less than 20 mL of water at 25 °C, such as monosaccharides, e. g. glucose, disaccharides, e. g. lactose, saccharose, oligosaccharides, e. g. dextrin, polysaccharides, sugar alcohols such as mannitol, sorbitol, maltitol, xylitol, lactitol, water-soluble pharmaceutical substances such as cellulose, e. g. microcrystalline cellulose or powdered cellulose, starch such as corn starch or its derivatives e. g. pregelatinized starch.

Binders used in the pharmaceutical formulation obtained according to the present invention are selected from the group of pharmaceutical substances which are water soluble and their solutions or melts have the ability to stick solid particles together. Non-limiting examples of such substances are cellulose ethers such as hydroxypropyl methylcellulose, hydroxy propylcellulose, povidone, copovidone, polyvinyl alcohol, graft copolymer of ethylene glycol and vinyl alcohol, polyethylene glycol with molecular mass between 1,000 and 20,000, Poloxamer, water-soluble pharmaceutical substances with molecular mass lower than 2,000 and melting point temperature and softening point temperature respectively lower than 150 °C such as macrogolglycerides, esters of saccharose with fatty acids having 10 to 22 carbon atoms.

Disintegrants used in the pharmaceutical formulation obtained according to the present invention are selected from the group of super-disintegrants such as metal salts of cross-linked carboxymethyl cellulose, cross-linked povidone (Polyplasdone or Kollidon CL), ion-exchange resins e. g. polacrylinate potassium, low-substituted hydroxypropyl cellulose, starch and its derivatives, microcrystalline cellulose.

As a glidant used in the pharmaceutical formulation obtained according to the present invention colloidal silicon dioxide can be selected.

Lubricants used in the pharmaceutical formulation obtained according to the present invention are selected from the group of metal salts of fatty acids such as magnesium stearate, stearic acids, sodium stearyl fumarate.

Stabilisers used in the pharmaceutical formulation obtained according to the present invention are selected from the group of pharmaceutical substances, which can adjust pH of the micro-environment to a value between 4 to 9.

Surfactants used in the pharmaceutical formulation obtained according to the present invention are selected from the group of surface active substances of non-ionic, anionic or amphoteric structure. Among non-ionic surfactants those with HLB value higher than 7, preferably higher than 9 are especially suitable, such as span derivatives e. g. polysorbates, esters of saccharose with fatty acids, poloxamers, derivatives of macrogol glycerides. Among anionic surfactants sodium doccusate, alkaline salts of ester of sulphuric (VI) acid with fatty alcohols having 10 to 24 carbon atoms such as sodium lauryl sulfate are especially suitable.

Plasticizers used in the pharmaceutical formulation obtained according to the present invention are selected from the group of polyethylene glycols with molecular mass lower than 10,000, citronic acid esters, esters of glycerol with fatty acids having 10 to 24 carbon atoms, propylene glycol.

Pigments used in the pharmaceutical formulation obtained according to the present invention are selected from the group of water insoluble metal oxides, such as ferrous oxides, titanium dioxide.

Colourants used in the pharmaceutical formulation obtained according to the present invention are selected from the group of water-soluble coloured pharmaceutically acceptable substances.

Antiadhesives used in the pharmaceutical formulation obtained according to the present invention are selected from the group of partial esters of glycerol with fatty acids such as glycerol monostearate, inorganic silicates such as talc.

Soluble means that 1 g is dissolved in less than 20 mL water at 25 °C in less than 2 hours at constant or periodic stirring.

Fast disintegrating means that it disintegrates in the presence of water or water dispersions to smaller particles in less than 20 minutes.

The manufactured pharmaceutical form can be filled into capsules or sachets or it already represents the final pharmaceutical form, i. e. coated tablets. In a special example of the present invention coated pellets can be mixed with excipients and pressed into tablets, which are administered orally, for example as orodispersible tablets which disintegrate into pellets in the mouth in less than 3 minutes, or perorally, for example as tablets, which disintegrate into pellets in the stomach, wherein disintegration time of the tablets into pellets is between 0.5 and 30 minutes, preferably 1 to 15 minutes. The content (amount) of pellets in the mixture for tabletting is preferably in the range 25 m./m.% to 75 m./m.%, preferably 35 m./m.% to 65 m./m.%. In the case of orodispersible tablets also substances for the correction of taste and flavour such as sweeteners and aromatizing agents can be used in addition to other excipients.

The final pharmaceutical form such as capsules or tablets, including orodispersible tablets and coated tablets are packed into primary pharmaceutical packaging material known from prior art. Preferred is the use of packaging material with low moisture and/or oxygen permeability.

The present invention pertains to subject-matter covered by the appended claims, in particular by combining two or more preferred embodiments as described herein. In other words, combinations of two or more preferred features are particularly preferred.

In the present application, unless specified otherwise, indications in chemical and enantiomeric purity refer to purity indications as determined by HPLC. The yield of chemical reactions is indicated in mol%. Other %age indications are wt-%, unless specified otherwise.

The invention is explained, yet not limited in any way, by the following examples.

### Example 1: Synthesis of crude duloxetine chloride

NaH (60 % in paraffin oil, 2.92 g) was suspended in DMSO (75 mL). After mixing of hydride (about 10 minutes) (S)-3-methyl-amino-1-(2-thienyl)-1-propanol (10g) at 20 °C was added carefully. Stirring was started slowly and the reaction mixture was heated to 30-40 °C. A solution of F-naphthalene (9 mL) in DMSO (10 mL) was then added dropwise in about 30 minutes. Red-brownish suspension was heated to 40-50 °C for 9 to 18 hours. Heating was stopped, the mixture was cooled to 23 °C and quenched with a slow dropwise addition into ice-cold solution of water (200 mL) and acetic acid (12 mL). It was heated to 20 °C (pH ≈ 5-5.5) and hexane (80 mL) was added. After extraction the phases were separated and the aqueous phase was alkalized with 50 % NaOH (12 mL). After complete addition of hydroxide iPrOAc (100mL) was added to the aqueous phase and during vigorous stirring the two-phase system was heated to 20 °C. The phases were separated and the aqueous phase was extracted one more time with iPrOAc (100 mL). The combined organic phases were rinsed 2X with water (2 X 80 mL) and treated for 30 minutes in hot with activated carbon. It was hot filtered and the filtrate was cooled. Into the cooled extract acetic acid (5 mL, 15±5°C) was added and the product was precipitated slowly with 2.5M HCl (23mL) in iPrOAc under vigorous stirring. The white suspension was stirred for 5 hours, cooled to 0-5 °C. It was filtered off and the filtrate was rinsed with iPrOAc. The product was dried in a vacuum drier for 5 hours. Obtained were 16.33 g (84 %) of crude duloxetine chloride (HPLC purity 99.87 %; 99.0 % ee), which was recrystallized from iPrOH. 15.18 g (93 %) of pure product (HPLC purity 99.94 % and 99.9 % ee) were obtained.

### Example 2: Synthesis of crude duloxetine chloride

Into a suspension of DMSO (75 mL) and NaH (60 % suspension in mineral oil; 2.9 g) (S)-(-)-N,N-dimethyl-3-hydroxy-3-(2-thienyl)propanamine was added carefully at 20-25 °C. The reaction mixture was then heated to 40 °C and a solution of F-naphthalene (9 mL) in DMSO (10 mL) was slowly added dropwise. After complete addition the suspension was heated to 50 °C and kept at 50 °C over night. After 18 hours the mixture was cooled to 20 °C and carefully poured into the ice-cold mixture of water (200 mL) and acetic acid (12 mL). After quenching the mixture was heated to 20 °C, pH was measured (5-5.5) and hexane (80 mL) was added. After 10 minutes of stirring the phases were separated. The aqueous phase was alkalized with 10 % NaOH and extracted into toluene (100 mL). The toluene phase was rinsed with water (1 x 60 mL). The toluene phase of (S)-N,N-dimethyl-3-(naphthalen-1-yloxy)-3-(thiophen-2-yl)propan-1-amine was heated to 55 °C, diisopropylethylamine (0.69 mL) was added and phenyl chlorofomiate (10.35 mL) was added slowly dropwise. It was heated for 1.25 hours, 1 % NaHCO₃ (215 mL) was added and after 10 minutes at 40-50 °C the phases were separated. The organic phase was rinsed with 0.5 M HCl (2 X 50 mL) and 1 % NaHCO₃ (50 mL). The rinsed toluene phase was concentrated on a rotary evaporator to a constant mass, dissolved in DMSO (150 mL), heated to 45 °C, the aqueous solution (50 mL) of NaOH (8.7 g) was added and heated for 18 hours at 50 °C. After the hydrolysis was completed the cooled mixture was poured into an ice-cold mixture of water (250 mL) and acetic acid (12 mL, pH about 5-5.5). The aqueous phase was rinsed with hexane (40 mL), alkalized with a 10 % base and a duloxetine base was extracted into isopropyl acetate (2 X 60 mL). The organic phase was rinsed with water (2 x 40 mL), treated in hot with activated carbon and the hot filtrate was cooled. To the cooled filtrate acetic acid (5 mL) was added and the product was precipitated with 2.5 M HCl in isopropyl acetate (23 mL). The suspension was stirred at 10 °C - 15 °C for 5-6 hours. The precipitate was filtered off and rinsed with cold isopropyl acetate. The product was dried in a vacuum drier at 50 °C for 5 hours. 13.5 g (75%) with 99.71 % HPLC purity and 99 % ee were obtained, which was recrystallized from iPrOH. 12.46 g (92%) of pure product (HPLC purity 99.92 % and 99.9 % ee) were obtained.

### Example 3: Precipitation of crude duloxetine chloride from isopropyl acetate

Duloxetine base (8 g, HPLC purity 96.4 % and 80.2 % ee) was dissolved in isopropyl acetate (110 mL), cooled and the product was slowly precipitated with 2.5M HCl (10 mL) in iPrOAc under vigorous stirring. The white suspension cooled to 0-5 °C was stirred for at least 5 hours. It was filtered, the filtrate was rinsed with iPrOAc. The product was dried in a vacuum drier for 5 hours. 6.5 g (72 %) of crude duloxetine chloride (HPLC purity 99.34 %; 84.4 % ee) were obtained.

### Example 4: Precipitation of crude duloxetine chloride from a mixture of acetic acid and isopropyl acetate

Duloxetine base (8 g, HPLC purity 96.4 % and 80.2 % ee) was dissolved in isopropyl acetate (110 mL), acetic acid (4 mL) was added, the obtained mixture was cooled and the product was slowly precipitated with 2.5M HCl (10 mL) in iPrOAc under vigorous stirring. The white suspension cooled to 0-5 °C was stirred for at least 5 hours. It was filtered, the filtrate was rinsed with iPrOAc. The product was dried in a vacuum drier for 5 hours. 6.9 g (77 %) of crude duloxetine chloride (HPLC purity 99.93 %; 99.0 % ee) were obtained.

### Example 5: Recrystallization of duloxetine chloride from isopropanol

2 g of crude duloxetine chloride (HPLC 89.63 %, 80 % ee) were suspended in isopropanol (15 mL). It was heated to a boiling point and the hot solution was filtered off and cooled within 3 hours to 0 °C. The precipitated product was filtered and the precipitate was rinsed with isopropanol. A product of purity 97.50 % and 97.2 % ee was obtained.

### Example 6: Recrystallization of duloxetine chloride from a mixture of acetic acid/isopropanol

2 g of duloxetine hydrochloride (HPLC 89.63 %; 80 % ee) were dissolved in acetic acid (2 mL) at 40 °C. Into the solution heated to 40 °C isopropanol (15 mL) was added dropwise. The precipitated product nucleated during vigorous stirring and the suspension was cooled to 0 °C within 3 hours, filtered and the precipitate was rinsed with isopropanol. A product of purity 98.60 % and 99.4 % ee was obtained.

### Example 7: Recrystallization of duloxetine chloride from a mixture of formic acid/isopropanol

The process is preformed in the same manner as in Example 6 with the exception that recrystallization is performed from a mixture of formic acid/isopropanol.

### Example 8: Recrystallization of duloxetine chloride from a mixture of propanoic acid/isopropanol

The process is preformed in the same manner as in Example 6 with the exception that recrystallization is performed from a mixture of propanoic acid/isopropanol.

### Example 9: Recrystallization of duloxetine chloride from a mixture of butanoic acid/isopropanol

The process is preformed in the same manner as in Example 6 with the exception that recrystallization is performed from a mixture of butanoic acid/isopropanol.

### Example 10: Recrystallization of duloxetine chloride from a mixture of acetic acid/ethyl acetate

3 g of duloxetine chloride was dissolved in acetic acid (3 mL) at 40 °C. Into the solution heated to 40 °C ethyl acetate (30 mL) was added dropwise. The precipitated product nucleated during vigorous stirring and the suspension was cooled to 0 °C within 3 hours, filtered and the precipitate was rinsed with ethyl acetate. After drying in a vacuum dryer at 50 °C for 5 hours 2.55 g (85 %) of pure duloxetine chloride was obtained.

### Example 11: Recrystallization of duloxetine chloride from a mixture of acetic acid/t-butylmethyl ether

2 g of duloxetine chloride was dissolved in acetic acid (2 mL) at 40 °C. Into the solution heated to 40 °C *t*-butylmethyl ether (40 mL) was added dropwise. The precipitated product nucleated during vigorous stirring and the suspension was cooled to 0 °C within 3 hours, filtered and the precipitate was rinsed with *t*-butylmethyl ether. After drying in a vacuum dryer at 50 °C for 5 hours 1.90 g (95 %) of pure duloxetine chloride was obtained.

### Example 12: Recrystallization of duloxetine chloride from a mixture of acetic acid /acetone

2 g of duloxetine chloride was dissolved in acetic acid (3 mL) at 40 °C. Into the solution heated to 40 °C acetone (25 mL) was added dropwise. The precipitated product nucleated during vigorous stirring and the suspension was cooled to 0 °C within 3 hours, filtered and the precipitate was rinsed with acetone. After drying in a vacuum dryer at 50 °C for 5 hours 1.3 g (65 %) of pure duloxetine chloride was obtained.

### Example 13: Pharmaceutical formulation (1)

| **Composition** | **Composition** |
|---|---|
| **Core** | |
| Neutral pellets (saccharose-starch) | 70.00 mg |
| Duloxetine chloride | 67.35 mg |
| Saccharose | 8.00 mg |
| Hydroxypropyl methylcellulose | 12.00 mg |

| **Intermediate coating** | |
|---|---|
| Hydroxypropyl methylcellulose | 14.00 mg |
| Talc | 24.00 mg |

| **Gastro-resistant coating** | |
|---|---|
| Hydroxypropyl methylcellulose acetate succinate | 64.47 mg |
| Talc | 19.34 mg |
| Sodium lauryl sulfate | 1.93 mg |
| Ammonia 25 % | q.s. |

| **Outer coating** | |
|---|---|
| Hydroxypropyl methylcellulose | 10.00 mg |
| Talc | 14.00 mg |
| Titanium dioxide | 7.00 mg |
| **Total** | **312.09 mg** |

Coating of duloxetine chloride was performed in a fluid-bed processor with a bottom-spray technique (Glatt GPCG-3) with a batch size of 1.5 kg. Duloxetine chloride was suspended in an aqueous solution of saccharose and hydroxypropyl methylcellulose. The suspension was slowly sprayed onto saccharose-starch granules and the cores so prepared were dried. In the next phase an intermediate coating was sprayed onto cores (neutral pellets coated with a coating containing the medicinal active ingredient). The process of coating with a dispersion of talc in an aqueous solution of hydroxypropyl methylcellulose was carried out on the same equipment as the previous phase. The coated pellets with the intermediate coating were finally dried to the content of residual moisture (determined by halogen drier Mettler HR 73, at 85 °C, 20 min) less than 1.6 m/m%.

A dispersion for the gastro-resistant coating was prepared by dispersing hydroxypropyl methylcellulose acetate succinate, talc, sodium lauryl sulfate and 25 m/vol. % of aqueous solution of ammonia in purified water. The prepared dispersion was sprayed onto pellets coated with the intermediate coating in the fluid-bed coater (the same as in the previous phases, i. e. Glatt GPCG-3). The operation of spraying was stopped after a specific amount of total liquid was sprayed and then drying was carried out in the coater. Gastro-resistant pellets were then coated also with an outer layer: in the coater (Glatt GPCG-3) an aqueous dispersion of hydroxypropyl methylcellulose, titanium dioxide and talc was sprayed onto floating pellets coated with the gastro-resistant coating, and after the coating process was completed the pellets were dried. The pellets were filled into gelatine capsules.

### Example 14: Pharmaceutical formulation (2)

| **Composition** | **Composition** |
|---|---|
| **Core** | |
| Neutral pellets (saccharose-starch) | 70.00 mg |
| Duloxetine chloride | 67.35 mg |
| Saccharose | 8.00 mg |
| Hydroxypropyl methylcellulose | 12.00 mg |

| **Intermediate coating** | |
|---|---|
| Hydroxypropyl methylcellulose | 12.00 mg |
| Talc | 19.20 mg |
| Sodium acetate | 1.68 mg |
| Saccharose | 8.00 mg |

| **Gastro-resistant coating** | |
|---|---|
| Copolymer of methacrylic acid and ethylacrylate 1:1 | 55.50 mg |
| Talc | 16.65 mg |
| Macrogol 6000 | 5.55 mg |
| Titanium dioxide | 5.55 mg |
| Polysorbate 80 | 2.50 mg |
| **Total** | **283.98 mg** |

Coating of duloxetine chloride was performed in a fluid-bed processor with a bottom-spray technique (Glatt GPCG-3) with a batch size of 1.5 kg. Duloxetine chloride was suspended in an aqueous solution of saccharose and hydroxypropyl methylcellulose. The suspension was slowly sprayed onto neutral pellets made of saccharose and corn starch. In the next phase an intermediate coating was sprayed onto cores. A process of coating with a dispersion of talc in an aqueous solution of hydroxypropyl methylcellulose was carried out on the same equipment as the previous phase. The coated pellets with the intermediate coating were finally dried to the content of residual moisture (determined by halogen drier Mettler HR 73, at 85 °C, 20 min) less than 1.6 m/m%.

The gastro-resistant coating dispersion containing the copolymer of methacrylic acid and ethyl acrylate, talc, Macrogol 6000, titanium dioxide and Polysorbate 80, was sprayed onto pellets coated with the intermediate coating in a fluid-bed apparatus. After coating, drying was carried out in the same fluid-bed coater as used for coating. Drying was carried out until the loss in drying was < 1.5 m/m% (determined by halogen dryer Mettler HR 73, at 85 °C, 20 min). The gastro-resistant pellets were filled into gelatine capsules.

### Example 15: Pharmaceutical formulation (3)

| **Composition** | **Composition** |
|---|---|
| **Core** | |
| Neutral pellets (saccharose-starch) | 70.00 mg |
| Duloxetine chloride | 67.35 mg |
| Saccharose | 8.00 mg |
| Hydroxypropyl methylcellulose | 12.00 mg |

| **Intermediate coating** | |
|---|---|
| Hydroxypropyl methylcellulose | 14.00 mg |
| Talc | 24.00 mg |

| **Gastro-resistant coating** | |
|---|---|
| Hydroxypropyl methylcellulose acetate succinate | 64.47 mg |
| Triethyl citrate | 6.45 mg |
| Talc | 19.34 mg |
| Sodium lauryl sulfate | 0.64 mg |
| Ammonia 25 % | q.s. |

| **Outer coating** | |
|---|---|
| Hydroxypropyl methylcellulose | 10.00 mg |
| Talc | 14.00 mg |
| Titanium dioxide | 7.00 mg |
| **Total** | **317.25 mg** |

Coating of duloxetine chloride was performed in a fluid-bed processor with a bottom-spray technique (Glatt GPCG-3) with a batch size of 1.5 kg. Duloxetine chloride was suspended in an aqueous solution of saccharose and hydroxypropyl methylcellulose. The suspension was slowly sprayed onto saccharose-starch granules and the cores so prepared were dried. In the next phase an intermediate coating was sprayed onto cores (coated neutral pellets with the coating containing the medicinal active ingredient). The process of coating with a dispersion of talc in an aqueous solution of hydroxypropyl methylcellulose was carried out on the same equipment as the previous phase. The coated pellets with the intermediate coating were finally dried to the content of residual moisture (determined by halogen drier Mettler HR 73, at 85 °C, 20 min) less than 1.6 m/m%.

A dispersion for gastro-resistant coating was prepared by dispersing hydroxypropyl methylcellulose acetate succinate, triethyl citrate, talc, sodium lauryl sulfate and 25 m/vol. % of aqueous solution of ammonia in purified water. The prepared dispersion was sprayed onto the pellets coated with intermediate coating in a fluid-bed coater (the same as in the previous phases, i. e. Glatt GPCG-3). The operation of spraying was stopped after a specific amount of total liquid was sprayed and then drying was carried out in a coater. The gastro-resistant pellets were then coated also with an outer layer: in the coater (Glatt GPCG-3), floating pellets, coated with the gastro-resistant coating, an aqueous dispersion of hydroxypropyl methylcellulose, titanium dioxide and talc was sprayed and after the coating process was completed the pellets were dried. The pellets were filled into gelatine capsules.

### Example 16: Pharmaceutical formulation of orodispersible tablets with duloxetine chloride

| **Tabletting mixture** | LP-16A | LP-16B | LP-16C |
|---|---|---|---|
| Pellets (composition according to Example 13) | 312.1 mg | 312.1 mg | 312.1 mg |
| Granulate (Macrogol 6000/MCC PH101= 1:4 | 150.0 mg | 178.30 mg | 50.0 mg |
| Mannitol | 75.0 mg | 70.0 mg | / |
| Lactose | 85.0 mg | 85.0 mg | / |
| Xylitol | / | / | 50.0 mg |
| MCC 101 | / | / | 140.0 mg |
| MCC 102 | / | / | 89.9 mg |
| Aerosil 200 | 3.1 mg | 3.0 mg | 3.0 mg |
| Corn starch | 14.8 mg | 14.6 mg | / |
| Talc | 4.0 mg | 4.0 mg | 4.0 mg |
| Magnesium stearate | 4.0 mg | 4.0 mg | 4.0 mg |
| Flavour (strawberry) | 6.1 mg | 6.1 mg | 6.0 mg |
| Flavour (cream) | 2.0 mg | 2.1 mg | 2.0 mg |
| Potassium acesulfamate | 3.1 mg | 3.0 mg | 3.0 mg |
| Polyplasdone XL (cross-linked povidone) | 80.0 mg | 80.5 mg | 78.0 mg |
| Tartaric acid | 4.0 mg | 4.0 mg | 4.0 mg |
| Copovidone VA 64 | / | / | 20.00 mg |

Granulate was prepared by means of extrusion at a temperature of 70 °C. The mixture of powder components without Aerosil and magnesium stearate was homogeneously mixed and admixed to the mixture of pellets and granulate in a biconical mixer. The mixture was then tabletted. Disintegration time according to Ph. Eur is < 1 minute

### Example 17: Pharmaceutical formulation (4)

| **Ingredients** | **Composition** |
|---|---|
| **Active core** | |
| Sucrose-starch nonpareils | 70,00 mg |
| Duloxetine hydrochloride | 67,35 mg |
| Hydroxypropyl methylcellulose | 12,00 mg |
| Sucrose | 8,00 mg |

| **Separating layer** | |
|---|---|
| Hydroxypropyl methylcellulose | 12,00 mg |
| Talc | 19,20 mg |
| Lactose monohydrate | 4,00 mg |
| Fructose | 4,00 mg |

| **Gastro-resistant coating** | |
|---|---|
| Hydroxypropyl methylcellulose phthalate HP-55 | 58,31 mg |
| Talc | 23,32 mg |
| Macrogol 6000 | 5,83 mg |
| Titanium dioxide | 2,92 mg |
| **Total** | **286,93 mg** |

The duloxetine layering was performed in a fluid bed processor using bottom spray technique (Wurster column) at a batch size of 1.5 kg. The duloxetine was suspended in the water solution of hydroxypropyl methylcellulose and sucrose. The suspension was slowly sprayed onto the agitating batch of beads. The prepared core material was dried and covered with separating layer in a Wurster column with a water solution of hydroxypropyl methylcellulose containing talc, lactose monohydrate and fructose.

The enteric coating suspension was prepared using hydroxypropyl methylcellulose phthalate HP-55, Macrogol 6000, talc and titanium dioxide. Hydroxypropyl methylcellulose phthalate HP-55 was dissolved in the ethanol/water mixture with weight ratio 8:2. Afterwards the water solution of Macrogol 6000 and suspension of talc and titanium dioxide were added. The final suspension was sprayed onto the pellets covered with separating layer in a fluid bed apparatus equipped with Wurster column. The spray operation was stopped when the specified amount of bulk liquid had been sprayed, and then drying was carried in the Wurster column. The pellets were filled into #1 gelatine capsules.

### Example 18: Pharmaceutical formulation (5)

| **Ingredients** | **Composition** |
|---|---|
| **Active core** | |
| Sucrose-starch nonpareils | 70,00 mg |
| Duloxetine hydrochloride | 67,35 mg |
| Hydroxypropyl methylcellulose | 12,00 mg |
| Sucrose | 8,00 mg |

| **Separating layer** | |
|---|---|
| Hydroxypropyl methylcellulose | 14,00 mg |
| Talc | 24,00 mg |

| **Gastro-resistant coating** | |
|---|---|
| Hydroxypropyl methylcellulose phthalate HP-55 | 34,01 mg |
| Hydroxypropyl methylcellulose phthalate HP-50 | 34,01 mg |
| Talc | 20,41 mg |
| Macrogol 6000 | 6,80 mg |
| Titanium dioxide | 3,40 mg |
| **Total** | **293,98 mg** |

The duloxetine layering was performed in a fluid bed processor using bottom spray technique (Wurster column) at a batch size of 1.5 kg. The duloxetine was suspended in the water solution of hydroxypropyl methylcellulose and sucrose. The suspension was slowly sprayed onto the agitating batch of beads. The prepared core material was dried and covered with separating layer in a Wurster column with a water solution of hydroxypropyl methylcellulose containing talc.

The enteric coating suspension was prepared using hydroxypropyl methylcellulose phthalate HP-50 and HP-55, Macrogol 6000, talc and titanium dioxide. Hydroxypropyl methylcellulose phthalate HP-50 and HP-55 were dissolved in the ethanol/water mixture with weight ratio 8:2. Afterwards the water solution of Macrogol 6000 and suspension of talc and titanium dioxide were added. The final suspension was sprayed onto the pellets covered with separating layer in a fluid bed apparatus equipped with Wurster column. The spray operation was stopped when the specified amount of bulk liquid had been sprayed, and then drying was carried in the Wurster column. The pellets were filled into #1 gelatine capsules.

## Claims

1. A process for the preparation of pharmaceutically acceptable salts of duloxetine according to the following scheme: 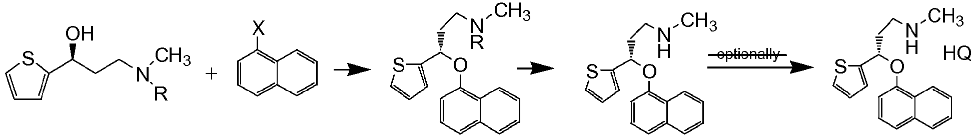
wherein R represents H or CH₃, and
wherein X represents a leaving group,
wherein Q represents a pharmaceutically acceptable anion, and
wherein the step of converting the R-substituted duloxetine into duloxetine is carried out only if R represents CH₃, **characterized in that** it comprises the following reaction step:
conversion of duloxetine into a pharmaceutically acceptable duloxetine salt with an acid, wherein the acid is selected such that it provides the anion of the target duloxetine salt, in an organic solvent selected from the group comprising ethers, esters, ketones, hydrocarbons and mixtures thereof, and wherein the conversion is carried out in the presence of an organic acid.

2. A process for the preparation of pharmaceutically acceptable salts of duloxetine according to claim 1, wherein X represents a leaving group selected from the group comprising F, Cl, Br and I.

3. A process for the preparation of pharmaceutically acceptable salts of duloxetine according to claim 1 or 2, wherein Q represents chloride.

4. A process for the preparation of pharmaceutically acceptable salts of duloxetine according to claim 1, 2 or 3, wherein the organic solvent is isopropyl acetate and/or wherein the organic acid is selected from the group comprising formic acid, acetic acid, propanoic acid, butanoic acid or any mixtures thereof.

5. A process for the preparation of pharmaceutically acceptable salts of duloxetine according to any one of claims 1 to 4, **characterized in that** it comprises the following reaction step:
recrystallization of a pharmaceutically acceptable duloxetine salt in a mixture of one or more organic solvents, selected from the group comprising ethers, esters, alcohols, hydrocarbons, and an organic acid.

6. A process for the preparation of pharmaceutically acceptable salts of duloxetine according to claim 5, wherein the one or more organic solvents comprise isopropanol and/or wherein the organic acid is selected from the group comprising formic acid, acetic acid, propanoic acid, butanoic acid or any mixtures thereof.

7. A process for the preparation of chemically and optically pure pharmaceutically acceptable salts of duloxetine according to any one of claims 1 to 6, **characterized in that**
a reaction between (S)-3-methyl-amino-1-(2-thienyl)-1-propanol and 1-fluoronaphthalene is performed as a first reaction step at a temperature between 30 °C and 90 °C.

8. A process for the preparation of chemically and optically pure pharmaceutically acceptable salts of duloxetine according to claim 7, wherein the reaction between (S)-3-methyl-amino-1-(2-thienyl)-1-propanol and 1-fluoronaphthalene is performed as a first reaction step at a temperature between 40 °C and 50 °C.

9. A process for the preparation of chemically and optically pure pharmaceutically acceptable salts of duloxetine according to any one of claims 1 to 8, **characterized in that**
a reaction between (S)-3-dimethyl-amino-1-(2-thienyl)-1-propanol and 1-fluoronaphthalene is performed as a first reaction step at a temperature between 30 °C and 90 °C.

10. A process for the preparation of chemically and optically pure pharmaceutically acceptable salts of duloxetine according to claim 9, **characterized in that** the reaction between (S)-3-dimethyl-amino-1-(2-thienyl)-1-propanol and 1-fluoronaphthalene is performed as a first reaction step at a temperature between 55 °C and 65 °C.

11. A process for the preparation of chemically and optically pure pharmaceutically acceptable salts of duloxetine according to any one of claims 1 to 6, **characterized in that**
R represents CH₃ and the complete process is carried out without isolation of intermediate products.

12. A process for the preparation of chemically and optically pure pharmaceutically acceptable salts of duloxetine according to any one of the preceding claims, **characterized in that**
the step of formation of a duloxetine salt is carried out with an excess of an acid up to 1.5 equivalents, wherein the indicated relative amount specifies the amount of the acid providing the anion for the duloxetine salt to be formed.

13. A process for the preparation of chemically and optically pure pharmaceutically acceptable salts of duloxetine according to claim 12, wherein the step of formation of a duloxetine salt is carried out with an excess of an acid up to 1.1 equivalents.

14. Use of an organic acid, selected from the group comprising formic acid, acetic acid, propanoic acid, butanoic acid or any mixtures thereof, in the preparation of a pharmaceutically acceptable duloxetine salt with a salt-forming acid, wherein the acid is selected such that it provides the anion of the target duloxetine salt, and/or in the recrystallization of the formed duloxetine salt.

15. Process for the manufacture of a pharmaceutical formulation including the step of manufacturing a pharmaceutically acceptable salt of duloxetine according to the process of one or more of claims 1 to 14 and the step of using the obtained pharmaceutically acceptable salt of duloxetine in the manufacture of the pharmaceutical formulation, wherein the pharmaceutically acceptable salt of duloxetine has an enantiomeric purity of more than 99.7 %, and chemical purity of more than 99.5 %.

## Patentansprüche

1. Verfahren zur Herstellung pharmazeutisch annehmbarer Salze von Duloxetin gemäß dem folgenden Schema: 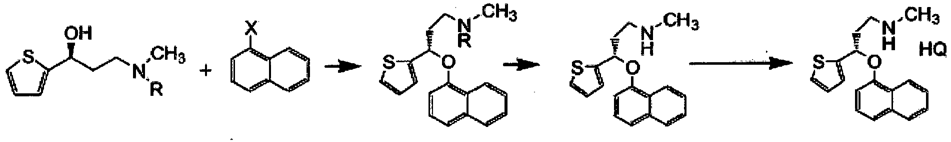
wobei R H oder CH₃ darstellt und
wobei X eine Abgangsgruppe darstellt,
wobei Q ein pharmazeutisch annehmbares Anion darstellt und
wobei der Schritt des Umsetzens des R-substituierten Duloxetins zu Duloxetin nur durchgeführt wird, wenn R CH₃ darstellt, **dadurch gekennzeichnet, dass** es den folgenden Reaktionsschritt umfasst:
Umsetzung von Duloxetin zu einem pharmazeutisch annehmbaren Duloxetinsalz mit einer Säure, wobei die Säure derart ausgewählt ist, dass sie das Anion des herzustellenden Duloxetinsalzes bereitstellt, in einem organischen Lösungsmittel, ausgewählt aus der Gruppe, umfassend Ether, Ester, Ketone, Kohlenwasserstoffe und Mischungen davon, und wobei die Umsetzung in der Anwesenheit einer organischen Säure durchgeführt wird.

2. Verfahren zur Herstellung pharmazeutisch annehmbarer Salze von Duloxetin gemäß Anspruch 1, wobei X eine Abgangsgruppe, ausgewählt aus der Gruppe, umfassend F, Cl, Br und I, darstellt.

3. Verfahren zur Herstellung pharmazeutisch annehmbarer Salze von Duloxetin gemäß Anspruch 1 oder 2, wobei Q Chlorid darstellt.

4. Verfahren zur Herstellung pharmazeutisch annehmbarer Salze von Duloxetin gemäß Anspruch 1, 2 oder 3, wobei das organische Lösungsmittel Isopropylacetat ist und/oder wobei die organische Säure ausgewählt wird aus der Gruppe, umfassend Ameisensäure, Essigsäure, Propansäure, Butansäure und beliebige Mischungen davon.

5. Verfahren zur Herstellung pharmazeutisch annehmbarer Salze von Duloxetin gemäß einem der Ansprüche 1 bis 4, **dadurch gekennzeichnet, dass** es den folgenden Reaktionsschritt umfasst:
Umkristallisation eines pharmazeutisch annehmbaren Duloxetinsalzes in einer Mischung von einem oder mehreren organischen Lösungsmitteln, ausgewählt aus der Gruppe, umfassend Ether, Ester, Alkohole, Kohlenwasserstoffe und eine organische Säure.

6. Verfahren zur Herstellung pharmazeutisch annehmbarer Salze von Duloxetin gemäß Anspruch 5, wobei das eine oder die mehreren organische/organischen Lösungsmittel umfasst/umfassen Isopropanol und/oder wobei die organische Säure ausgewählt wird aus der Gruppe, umfassend Ameisensäure, Essigsäure, Propansäure, Butansäure oder beliebige Mischungen davon.

7. Verfahren zur Herstellung von chemisch oder optisch reinen pharmazeutisch annehmbaren Salzen von Duloxetin gemäß einem der Ansprüche 1 bis 6, **dadurch gekennzeichnet, dass**
eine Reaktion zwischen (S)-3-Methyl-amino-1-(2-thienyl)-1-propanol und 1-Fluornaphthalin als ein erster Reaktionsschritt bei einer Temperatur zwischen 30°C und 90°C durchgeführt wird.

8. Verfahren zur Herstellung von chemisch und optisch reinen pharmazeutisch annehmbaren Salzen von Duloxetin gemäß Anspruch 7, wobei die Reaktion zwischen (S)-3-Methyl-amino-1-(2-thienyl)-1-propanol und 1-Fluornaphthalin als ein erster Reaktionsschritt bei einer Temperatur zwischen 40°C und 50°C durchgeführt wird.

9. Verfahren zur Herstellung von chemisch und optisch reinen pharmazeutisch annehmbaren Salzen von Duloxetin gemäß einem der Ansprüche 1 bis 8, **dadurch gekennzeichnet, dass**
eine Reaktion zwischen (S)-3-Dimethyl-amino-1-(2-thienyl)-1-propanol und 1-Fluornaphthalin als ein erster Reaktionsschritt bei einer Temperatur zwischen 30°C und 90°C durchgeführt wird.

10. Verfahren zur Herstellung von chemisch und optisch reinen pharmazeutisch annehmbaren Salzen von Duloxetin gemäß Anspruch 9, **dadurch gekennzeichnet, dass** die Reaktion zwischen (S)-3-Dimethyl-amino-1-(2-thienyl)-1-propanol und 1-Fluornaphthalin als ein erster Reaktionsschritt bei einer Temperatur zwischen 55°C und 65°C durchgeführt wird.

11. Verfahren zur Herstellung von chemisch und optisch reinen pharmazeutisch annehmbaren Salzen von Duloxetin gemäß einem der Ansprüche 1 bis 6, **dadurch gekennzeichnet, dass**
R CH₃ darstellt und das komplette Verfahren ohne Isolierung von Zwischenprodukten durchgeführt wird.

12. Verfahren zur Herstellung von chemisch und optisch reinen pharmazeutisch annehmbaren Salzen von Duloxetin gemäß einem der vorhergehenden Ansprüche, **dadurch gekennzeichnet, dass**
der Schritt der Bildung eines Duloxetinsalzes mit einem Überschuss an einer Säure von bis zu 1,5 Äquivalenten durchgeführt wird, wobei die angegebene relative Menge die Menge der Säure, die das Anion für das zu bildende Duloxetinsalz bereitstellt, spezifiziert.

13. Verfahren zur Herstellung von chemisch und optisch reinen pharmazeutisch annehmbaren Salzen von Duloxetin gemäß Anspruch 12, wobei der Schritt der Bildung eines Duloxetinsalzes mit einem Überschuss an einer Säure von bis zu 1,1 Äquivalenten durchgeführt wird.

14. Verwendung einer organischen Säure, ausgewählt aus der Gruppe, umfassend Ameisensäure, Essigsäure, Propansäure, Butansäure oder beliebige Mischungen davon, bei der Herstellung eines pharmazeutisch annehmbaren Duloxetinsalzes mit einer salzbildenden Säure, wobei die Säure derart ausgewählt ist, dass sie das Anion für das gewünschte Duloxetinsalz bereitstellt, und/oder bei der Umkristallisation des gebildeten Duloxetinsalzes.

15. Verfahren zur Herstellung einer pharmazeutischen Formulierung, umfassend den Schritt der Herstellung eines pharmazeutisch annehmbaren Salzes von Duloxetin gemäß dem Verfahren nach einem der Ansprüche 1 bis 14 und den Schritt der Verwendung des erhaltenen pharmazeutisch annehmbaren Salzes von Duloxetin bei der Herstellung der pharmazeutischen Formulierung, wobei das pharmazeutisch annehmbare Salz von Duloxetin eine Enantiomerenreinheit von mehr als 99,7% und eine chemische Reinheit von mehr als 99,5% aufweist.

## Revendications

1. Procédé de préparation de sels pharmaceutiquement acceptables de duloxétine selon le schéma suivant : 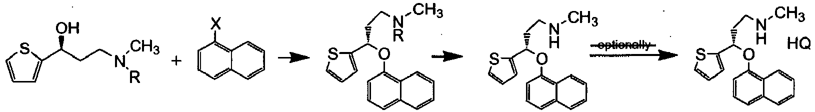
dans lequel R représente H ou CH₃, et
dans lequel X représente un groupe partant,
dans lequel Q représente un anion pharmaceutiquement acceptable, et
dans lequel l'étape de conversion de la duloxétine substituée par R en duloxétine est mise en oeuvre seulement si R représente CH₃, **caractérisé en ce qu'**il comprend l'étape réactionnelle suivante :
conversion de la duloxétine en un sel de duloxétine pharmaceutiquement acceptable avec un acide, dans lequel l'acide est sélectionné de sorte qu'il fournisse l'anion du sel de duloxétine cible, dans un solvant organique sélectionné dans le groupe comprenant les éthers, les esters, les cétones, les hydrocarbures et des mélanges de ceux-ci, et dans lequel la conversion est mise en oeuvre en présence d'un acide organique.

2. Procédé de préparation de sels pharmaceutiquement acceptables de duloxétine selon la revendication 1, dans lequel X représente un groupe partant sélectionné dans le groupe comprenant F, Cl, Br et I.

3. Procédé de préparation de sels pharmaceutiquement acceptables de duloxétine selon la revendication 1 ou 2, dans lequel Q représente un chlorure.

4. Procédé de préparation de sels pharmaceutiquement acceptables de duloxétine selon la revendication 1, 2 ou 3, dans lequel le solvant organique est l'acétate d'isopropyle et/ou dans lequel l'acide organique est sélectionné dans le groupe comprenant l'acide formique, l'acide acétique, l'acide propanoïque, l'acide butanoïque ou tout mélange de ceux-ci.

5. Procédé de préparation de sels pharmaceutiquement acceptables de duloxétine selon l'une quelconque des revendications 1 à 4, **caractérisé en ce qu'**il comprend l'étape réactionnelle suivante :
recristallisation d'un sel de duloxétine pharmaceutiquement acceptable dans un mélange d'un ou plusieurs solvants organiques, sélectionnés dans le groupe comprenant les éthers, les esters, les alcools, les hydrocarbures, et un acide organique.

6. Procédé de préparation de sels pharmaceutiquement acceptables de duloxétine selon la revendication 5, dans lequel les un ou plusieurs solvants organiques comprennent de l'isopropanol et/ou dans lequel l'acide organique est sélectionné dans le groupe comprenant l'acide formique, l'acide acétique, l'acide propanoïque, l'acide butanoïque ou tout mélange de ceux-ci.

7. Procédé de préparation de sels pharmaceutiquement acceptables de duloxétine chimiquement et optiquement purs selon l'une quelconque des revendications 1 à 6, **caractérisé en ce que**
une réaction entre le (S)-3-méthyl-amino-1-(2-thiényl)-1-propanol et le 1-fluoronaphtalène est mise en oeuvre en tant que première étape réactionnelle à une température entre 30°C et 90°C.

8. Procédé de préparation de sels pharmaceutiquement acceptables de duloxétine chimiquement et optiquement purs selon la revendication 7, dans lequel la réaction entre le (S)-3-méthyl-amino-1-(2-thiényl)-1-propanol et le 1-fluoronaphtalène est mise en oeuvre en tant que première étape réactionnelle à une température entre 40°C et 50°C.

9. Procédé de préparation de sels pharmaceutiquement acceptables de duloxétine chimiquement et optiquement purs selon l'une quelconque des revendications 1 à 8, **caractérisé en ce que**
une réaction entre le (S)-3-diméthyl-amino-1-(2-thiényl)-1-propanol et le 1-fluoronaphtalène est mise en oeuvre en tant que première étape réactionnelle à une température entre 30°C et 90°C.

10. Procédé de préparation de sels pharmaceutiquement acceptables de duloxétine chimiquement et optiquement purs selon la revendication 9, **caractérisé en ce que** la réaction entre le (S)-3-diméthyl-amino-1-(2-thiényl)-1-propanol et le 1-fluoronaphtalène est mise en oeuvre en tant que première étape réactionnelle à une température entre 55°C et 65°C.

11. Procédé de préparation de sels pharmaceutiquement acceptables de duloxétine chimiquement et optiquement purs selon l'une quelconque des revendications 1 à 6, **caractérisé en ce que**
R représente CH3 et la totalité du procédé est mise en oeuvre sans isoler de produits intermédiaires.

12. Procédé de préparation de sels pharmaceutiquement acceptables de duloxétine chimiquement et optiquement purs selon l'une quelconque des revendications précédentes, **caractérisé en ce que**
l'étape de formation d'un sel de duloxétine est mise en oeuvre avec un excès d'un acide jusqu'à 1,5 équivalent, dans laquelle la quantité relative indiquée spécifie la quantité de l'acide fournissant l'anion pour le sel de duloxétine devant être formé.

13. Procédé de préparation de sels pharmaceutiquement acceptables de duloxétine chimiquement et optiquement purs selon la revendication 12, dans lequel l'étape de formation d'un sel de duloxétine est mise en oeuvre avec un excès d'un acide jusqu'à 1,1 équivalent.

14. Utilisation d'un acide organique, sélectionné dans le groupe comprenant l'acide formique, l'acide acétique, l'acide propanoïque, l'acide butanoïque ou tout mélange de ceux-ci, dans la préparation d'un sel de duloxétine pharmaceutiquement acceptable avec un acide formant un sel, dans laquelle l'acide est sélectionné de sorte qu'il fournisse l'anion du sel de duloxétine cible, et/ou dans la recristallisation du sel de duloxétine formé.

15. Procédé de fabrication d'une formulation pharmaceutique incluant l'étape de fabrication d'un sel pharmaceutiquement acceptable de duloxétine selon le procédé de l'une ou plusieurs des revendications 1 à 14 et l'étape d'utilisation du sel pharmaceutiquement acceptable de duloxétine obtenu dans la fabrication de la formulation pharmaceutique, dans lequel le sel pharmaceutiquement acceptable de duloxétine possède une pureté énantiomérique de plus de 99,7 %, et une pureté chimique de plus de 99,5 %.
